# EUROPÄISCHE PATENTSCHRIFT

(11) **EP 2 382 304 B1**
(45) Veröffentlichungstag und Bekanntmachung des Hinweises auf die Patenterteilung: **11.03.2020**
(21) Anmeldenummer: 10702430.9
(22) Anmeldetag: 27.01.2010
(51) Int. Cl.: C12M 1/107, C12M 1/113

(54) **FERMENTER-BESCHICKUNGSANLAGE FÜR VERGÄRBARE BIOMASSE EINER BIOGASANLAGE UND VERFAHREN ZUM BETRIEB DER BESCHICKUNGSANLAGE**
FERMENTER FEED SYSTEM FOR FERMENTABLE BIOMASS OF A BIOGAS SYSTEM AND METHOD FOR OPERATING THE FEED SYSTEM
INSTALLATION DE CHARGEMENT POUR FERMENTEUR POUR UNE BIOMASSE FERMENTABLE D'UNE INSTALLATION DE PRODUCTION DE BIOGAZ, ET PROCÉDÉ DE FONCTIONNEMENT DE CETTE INSTALLATION

(30) Priorität: 28.01.2009 EP 09001148
(43) Veröffentlichungstag der Anmeldung: 02.11.2011
(73) Patentinhaber: BTS Biogas Srl/GmbH, 39031 Bruneck (IT)
(72) Erfinder: Niederbacher, Michael, 39031 Bruneck (IT)
(74) Vertreter: Liebl, Thomas
(86) Internationale Anmeldenummer: PCT/EP2010/000497
(87) Internationale Veröffentlichungsnummer: WO 2010/086158

(56) Entgegenhaltungen:
- EP-A- 0 201 928
- EP-A- 0 555 792
- EP-A- 0 613 870
- EP-A- 0 781 742
- EP-A- 1 125 894
- EP-A- 1 978 086
- WO-A-2005/028375
- WO-A-2008/029163
- DE-A1- 3 844 700
- DE-A1-102007 029 700
- US-A1- 2005 026 262

## Beschreibung

Die Erfindung betrifft eine Fermenter-Beschickungsanlage für vergärbare Biomasse einer Biogasanlage sowie ein Verfahren zum Betrieb der Beschickungsanlage.

Bei allgemein bekannten Biogasanlagen wird zur Gewinnung von Biogas Biomasse mittels eines anaeroben Nassvergärungsprozesses vergoren. Zur Beschickung des Fermenters mit vergärbarer Biomasse werden üblicherweise automatisierte Fermenter-Beschickungsanlagen eingesetzt, wobei für flüssige Substratbestandteile Pumpen und für feste Substratbestandteile Förder- und Aufbereitungseinrichtungen zwischen einer Biomassenfeststofflagerstelle und einem Fermenter-Beschickungsanschluss vorgesehen sind. Eine solche Förder- und Aufbereitungseinrichtung umfasst regelmäßig eine Dosiervorrichtung sowie zum Fermenter führende Fördermittel, insbesondere Förderbänder und/oder Förderschnecken. Eine bekannte Dosiervorrichtung besteht beispielsweise aus einem mit Biomasse befüllbaren Container, der auf Wiegezellen steht, aus dem jeweils eine vorbestimmte Biomassenmenge durch einen Bodenförderer in Verbindung mit stirnseitigen Austragschnecken einem Förderband für einen Weitertransport zum Fermenter zugeführt wird.

Das erzeugte Biogas wird entweder nach einer Reinigung direkt in Gasleitungen eingespeist oder in einem Blockheizkraftwerk für die Stromerzeugung verwendet. Die wirtschaftliche Effektivität einer Biogasanlage hängt wesentlich von der Art der einsetzbaren Biomassen, deren Verfügbarkeit sowie der Effektivität des Fermentationsprozesses mit einer möglichst kurzzeitigen Substratverweilzeit im Fermenter bei hohem Ausfaulgrad für eine hohe Biogasausbeute ab.

Weiter ist ein Verfahren zur Herstellung von Pflanzsubstraten für die Aufzucht von Kulturpflanzen im Garten- und Landschaftsbau bekannt (DE 44 44 745 C1), bei dem Holzabfälle mit Klärschlamm, Gülleschlamm und Biokompost vorgemischt und in einem Schneckenextruder zerrieben, zerfasert und intensiv miteinander vermischt werden, wobei Temperaturen von über 100°C entstehen. Dadurch werden Krankheitserreger, Bakterien, etc. abgetötet, so dass am Extruderkopf weitgehend keimfreies Pflanzsubstrat zur weiteren Verwendung austritt. Weiter ist ein ähnliches Verfahren zur Entsorgung biologischer Abfälle von Städten und Gemeinden bekannt (DE 195 14 975 A1).

Aus der EP 1 978 086 A1 ist bereits ein Verfahren zum Hybridaufschluss von wasser- und lignocellulosehaltiger Biomasse durch Zerfaserung und Hydrolyse bekannt, bei der in einer ersten Stufe lignocellulosehaltige Biomasse durch ein Zerfaserungsgerät aufbereitet wird, anschließend in einer nachfolgenden Stufe die aufbereitete, lignosecellulosehaltige Biomasse in einer thermischen Hydrolyse in einer Hochdruckmikrowelle erwärmt und hydrolysiert wird, bevor schließlich in einer letzten Stufe die hydrolysierte lignocellulosehaltige Biomasse einem anaeroben Vergärungsprozess unterworfen wird. Im Rahmen der thermischen Hydrolyse der Biomasse in einer Hochdruckmikrowelle wird dabei die wasserhaltige Biomasse in kurzer Zeit auf weit über 100°C erhitzt, so dass durch die hohen Temperaturen und das verdampfte Wasser aus der Biomasse eine thermische Hydrolyse stattfindet. Die Temperaturerhöhung erfolgt dabei unter einem starken Druckanstieg von beispielsweise auf 5 bis 25 bar bis auf 160 bis 220°C. Die Zerfaserung der Biomasse erfolgt dabei bevorzugt mittels eines Extruders, der als Einfach- oder Doppelschneckenextruder bzw. Doppelkammerextruder ausgeführt sein kann. Mit einer derartigen Verfahrensführung soll neben der Erhöhung einer Biogasausbeute auch eine Verkürzung der bei Biogas üblichen Verweil- bzw. Vergärungszeiten im Fermenter eines Biogasreaktors erzielt werden. Ein derartiger Aufbau ist insbesondere durch den Einsatz einer Hochdruckmikrowelle relativ energieintensiv und auch herstellungstechnisch teuer.

Weiter zeigt die EP 0 613 870 A1 ein Verfahren für die Wiederverwertung von wasserhaltigem pflanzlichem Material, bei dem pflanzliches Material mit mechanischen Mitteln zerkleinert und zellulär aufgeschlossen wird. Konkret wird hier mit einem in einem oberen Bereich eines Behälters horizontal angeordnetem Rohr kontinuierlich oder stoßweise Wasser zugegeben. Die Steuerung der Wasserzugabe erfolgt mit Hilfe eines Motorventils. Das Rohr weist eine Mehrzahl von nach oben gerichteten Düsen oder Löchern auf, die für eine Verwirbelung des oberflächennahen Wasserbereichs sorgen sollen. Eine Kolloidmühle zerkleinert bzw. mahlt dann das nasse Schnittgut bis zur Zerstörung der Zellstrukturen. Der Antrieb der Kolloidmühle, der Antrieb einer Förderschnecke und der Antrieb eines Dosierbandes werden von einer gemeinsamen Steuerschaltung in aufeinander abgestimmter Weise geregelt.

Weiter offenbart die EP 0 781 742 A1 eine Aufbereitung zur Zerkleinerung und zum Mischen einer Extruderschnecke. Ein Bezug zu einem Fermenter einer Biogasanlage ist hier nicht offenbart.

Die WO 2005/028375 A1 offenbart ein Verfahren und eine Einrichtung zum Zerkleinern partikulärer organischer Substanzen in Suspensionen von Mikroorganismen in einem Trägermedium, insbesondere in Abwässern oder Schlämmen biologischer Kläranlagen, wobei die organischen Substanzen in einem Wirkraum Trägheitskräften in Folge kurzzeitiger extremer Beschleunigung und unmittelbar darauffolgender Verzögerung des in einem geschlossenen Strömungskanal strömenden Trägermediums ausgesetzt werden. Zum Erzeugen dieser Trägheitskräfte wird das Trägermedium in dem Wirkraum zum Verdampfen und nachfolgend zum Kondensieren gebracht, wobei diese Änderungen des Aggregatzustandes des Trägermediums durch Änderung der Strömungsgeschwindigkeit erfolgt.

Und schließlich offenbart die EP 1 125 894 A1 ein Verfahren zur Weiterverarbeitung von verunreinigtem, in einem Sammelbereich vorliegenden Biomüll sowie eine Vorrichtung zur Durchführung des Verfahrens. Der in einer Auffangwanne eingebrachte Biomüll wird in dieser dosiert und flächig verteilt in ein Wasserbecken verbracht, in welchem er aufgeschwämmt und von einer Zertrenneinrichtung zerteilt und weiter transportiert wird. Die gegenüber wasserspezifisch schwereren Anteile sinken dabei ab und werden über Sammel- und Austragsrinnen abgeführt. Die zusammen mit dem Restmüll aus der Wasserwanne austretenden, gegenüber wasserspezifisch leichteren Verunreinigungen werden gegebenenfalls über eine Abscheidevorrichtung abgeschieden. Der Restmüll wird einem Mahlwerk mit einem Mahlwerk sowie anschließend einer Schneckenpresse zugeführt. Das Pressgut wird in einem kompostierbaren und in einem fermentierbaren Anteil aufgetrennt und in Einrichtungen zum Fermentieren und Kompostieren weiterverarbeitet.

Aufgabe der Erfindung ist es daher, eine Fermenter-Beschickungsanlage und ein Verfahren zum Betreiben einer derartigen Fermenter-Beschickungsanlage vorzuschlagen, welche Beschickungsanlage baulich einfach aufgebaut ist und mit einem energetisch geringen Aufwand betrieben werden kann, wobei dieser Fermenter-Beschickungsanlage ein großes Spektrum von Biomassearten für eine Biogaserzeugung zugeführt und so aufbereitet werden kann, dass der anschließende Fermentationsprozess auch bei hohem Trockensubstanzgehalt mit kurzen Substratverweilzeiten und einem hohen Ausfaulgrad und damit hoher Biogasausbeute effektiv und funktionssicher ablaufen kann.

Diese Aufgabe wird hinsichtlich der Fermenter-Beschickungsanlage mit den Merkmalen des Anspruchs 1 und hinsichtlich des Verfahrens mit den Merkmalen des Anspruchs 11 gelöst.

Gemäß Anspruch 1 ist eine Fermenter-Beschickungsanlage für vergärbare Biomasse einer Biogasanlage vorgesehen, die eine Förder- und Aufbereitungseinrichtung zwischen einer Biomasselagerstelle, bevorzugt als befahrbares Fahrsilo ausgebildet, und wenigstens einem Fermenter, insbesondere wenigstens einem Fermenter-Beschickungsanschluss, aufweist, wobei die Förder- und Aufbereitungseinrichtung wenigstens ein Fördermittel für die dem Fermenter zuzuführende Biomasse aufweist. In die Förder- und Aufbereitungseinrichtung ist weiter eine vorzugsweise mechanische Zellaufschlussvorrichtung für Biomasse integriert, über die zumindest ein Teil der zum Fermenter förderbaren Biomasse geleitet und nach dem Zellaufschluss dem Fermenter zugeführt wird.

Durch einen solchen Zellaufschluss können vorteilhaft Biomassefeststoffe bei der Biogaserzeugung im Fermentationsprozess, insbesondere Stroh und Gras verwendet werden, die ansonsten nicht oder nur uneffektiv vergärbar sind. Weiter tritt in Verbindung mit dem Zellaufschluss eine Zerkleinerung der Biomassefeststoffe auf, so dass diese für die Bakterien im Fermenter und damit im Gärprozess besser zugängig sind bzw. eine leichter angreifbare Struktur ausbilden, so dass ein hoher Trockensubstanzgehalt im Substrat bei geringen Verweilzeiten mit hoher Biogasausbeute möglich ist. Zudem können dadurch bei der Dimensionierung und beim Betrieb der Fermenter-Rührtechnik Einsparungen erzielt werden, wobei eine Tendenz zur Bildung von Schwimmschichten oder Sinkschichten reduziert ist. Durch diese Vorbehandlung wird die Biogasausbeute der Biomasse somit erheblich gesteigert, was wiederum einen geringen Einsatz an Biomasse zur Folge hat und was sich wiederum in kleineren Baugrößen für die Fermenter wiederspiegelt, so dass die Biogasanlage in der Anschaffung und auch im Betrieb kostengünstiger ist als herkömmliche Biogasanlagen.

Erfindungsgemäß ist der Durchmesser einer Auslassöffnung der Zellaufschlussvorrichtung, bevorzugt der Durchmesser einer Auslassöffnung bzw. -düse eines Zellaufschluss-Extruders, durch ein gesteuert verstellbares Stellglied, insbesondere ein gesteuert motorisch mittels eines Elektroantriebs oder Hydraulikantriebs oder Pneumatikantriebs verstellbares Stellglied, veränderbar. Der Zellaufschlussvorrichtung ist ferner wenigstens ein Temperatursensor und/oder Drucksensor zugeordnet, mittels dem die Temperatur und/oder der Druck in einem vorgegebenen Bereich der Zellaufschlussvorrichtung und/oder der Biomasse erfasst wird. Weiter ist ein Durchmesser-Regler vorgesehen, dem das Ausgangssignal des Temperatursensors als Temperatur-Istwert und/oder des Drucksensors als Druck-Istwert zugeführt wird, wobei der Istwert mit einem vorgegebenen Sollwert verglichen wird und das Stellglied bei einem Absinken des Istwerts unter den Sollwert für eine definierte, vorgegebene Reduzierung des Auslassöffnungs-Durchmessers angesteuert wird.

Mit der erfindungsgemäßen Fermenter-Beschickungsanlage kann somit auf einfache und funktionssichere Weise ein gewünschter Zellaufschlussprozess sichergestellt werden bzw. die dazu erforderliche Temperatur und/oder der dafür erforderliche Druck in der Zellaufschlussvorrichtung sichergestellt werden. Konkret werden hierbei die Messwerte des Temperatursensors und/oder Drucksensors die zum Beispiel in der Nähe der Auslassdüse einer Zellaufschlussvorrichtung angeordnet sind, als Istwerte dem Durchmesser-Regler zugeführt, an dem eine Solltemperatur und/oder ein Solldruck für einen sicheren Zellaufschluss, insbesondere eine Solltemperaturvorgabe von mindestens 40°C, höchst bevorzugt von mindestens 60°C einstellbar ist. Bei einem tendenziellen Absinken des Isttemperaturwerts und/oder des Istdruckwerts unter die jeweils vorgegebenen Sollwerte wird dann vom Regler das Stellglied für eine Reduzierung des Auslassöffnungs-Durchmessers angesteuert. Dadurch wird dann bei entsprechend gleicher Leistungsaufnahme der Zellaufschlussvorrichtung mehr Energie in die aktuell darin geförderte und aufbereitete Biomasse eingebracht, so dass eine auf den jeweiligen Einzelfall abgestimmte optimale Biomasse für eine Vergärung in einem Fermenter einer Biogasanlage zur Verfügung gestellt werden kann.

Gemäß einer bevorzugten Ausführungsform wird nach Anspruch 2 vorgeschlagen, dass der Temperatursensor und/oder der Drucksensor in der Zellaufschlussvorrichtung unmittelbar vor oder in der Auslassöffnung, insbesondere unmittelbar vor oder in einer Auslassdüse eines Zellaufschlussextruders angeordnet wird. Beispielsweise kann vorgesehen sein, dass der Temperatursensor und/oder der Drucksensor an einem Wandbereich oder einem Innenauskleidungsbereich der Zellaufschlussvorrichtung auslassöffnungsnah angeordnet ist. Diese zuvor angegebenen beispielhaften Anbringungsmöglichkeiten der Sensoren sollen belegen, dass es verschiedene Möglichkeiten gibt im Bereich der Zellaufschlussvorrichtung eine Isttemperatur und/oder einen Istdruck zu erfassen, um darüber direkt oder indirekt abzuleiten, ob im Bereich der in der Zellaufschlussvorrichtung aufbereiteten Biomasse eine gewünschte, für einen optimierten Zellaufschluss geeignete Temperatur und/oder Druck vorliegt. Eine indirekte Erfassung der Temperatur bedeutet dabei, dass es zum Beispiel möglich ist, die Temperatur einem auslassöffnungsnahen Wandbereich der Zellaufschlussvorrichtung zu messen, woraus dann auf die Temperatur in der aufbereiteten Biomasse geschlossen werden kann.

Vorteilhaft wird die Zellaufschlussvorrichtung gemäß Anspruch 3 im Förderweg für die Biomasse zwischen einem Dosierer und dem Fermenter integriert.

Dadurch ist die Zellaufschlussvorrichtung im Außenbereich angeordnet und den Witterungseinflüssen ausgesetzt. Es wird daher mit Anspruch 4 vorgeschlagen, die Zellaufschlussvorrichtung wärmegedämmt auszuführen, entweder durch eine unmittelbare Wärmedämmung in der Art einer Wärmedämm-Umhüllung oder durch eine mittelbare Wärmedämmung in der Art einer Wärmedämm-Einhausung.

Eine solche Wärmedämmung ist nicht nur, aber zum Beispiel insbesondere in Verbindung mit den Merkmalen nach Anspruch 5 vorteilhaft und wesentlich, wenn ein Zellaufschlussextruder mit Schneckenwellen, vorzugsweise ein Doppelschneckenextruder, mit einer Zuführeinrichtung und einer Auslassdüse verwendet wird. Gegebenenfalls ist es aber auch ausreichend die Wärmedämmung nach einem Teilaspekt des Anspruchs 6 zum Beispiel nur im Bereich der Schneckenwellen vorzusehen. Der Zellaufschluss erfolgt bei einem derartigen Extruder durch die Einbringung mechanischer Energie in die im Extruder geförderte Biomasse durch die Friktion der mit den Schnecken rotierenden Biomasse an der stehenden Gehäusezylinderwand. Dabei können hohe Drücke und hohe Temperaturen in der Biomasse erzeugt werden. Beim Austreten der Biomasse an der Auslassdüse erfolgt dann eine Druckentspannung und/oder Temperaturabkühlung, die zum Zellaufschluss führt.

Mit den Merkmalen des Anspruchs 7 wird eine vorteilhafte Ausbildung des Förderwegs mit integrierter Zellaufschlussvorrichtung, insbesondere mit einem Zellaufschlussextruder, beansprucht. Dabei führt ein erstes Fördermittel, vorzugsweise ein erster Bandförderer, von der Dosiervorrichtung in einen Bereich oberhalb der Zellaufschlussvorrichtung. Ein zweites Fördermittel, vorzugsweise ein zweiter Bandförderer, führt ausgehend von einem Bereich unter der Auslassöffnung bzw. -düse der Zellaufschlussvorrichtung zum Fermenter. Mit dem ersten Fördermittel kann nun direkt Biomasse der Zellaufschlussvorrichtung, zum Beispiel über einen Zuführtrichter mit zum Beispiel einer motorisch antreibbaren Stopfschnecke, zugeführt werden. Um bei einer Störung an der Zellaufschlussvorrichtung, beispielsweise durch harte Störstoffe, insbesondere Metallstücke, eine weitere Beschickung des Fermenters sicherzustellen, ist bevorzugt eine betätigbare Stellklappe vorgesehen, mit der entweder je nach Stellposition die Zellaufschlussvorrichtung beschickt oder ein Bypassweg unmittelbar zum zweiten Fördermittel für einen direkten Transport zum Fermenter ermöglicht wird.

Alternativ dazu ist nach Anspruch 8 im Anschluss an das erste Fördermittel ein drittes Fördermittel vorgesehen, dessen Laufrichtung umsteuerbar ist, so dass damit in der einen Laufrichtung die Zellaufschlussvorrichtung beschickt werden kann, während die Biomasse in der anderen Laufrichtung, beispielsweise über einen Rohrstutzen, unmittelbar in einem Direkt- und Bypassweg zum zweiten Fördermittel transportierbar ist. Bei dieser Ausführungsform mit einem dritten Fördermittel, insbesondere Bandförderer, ist die vorstehend erläuterte Stellklappe in Verbindung mit dem dortigen Bypassweg entbehrlich.

Zum Schutz der Zellaufschlussvorrichtung, insbesondere eines Zellaufschluss-Extruders, und für eine verbesserte Betriebssicherheit wird mit Anspruch 9 vorgeschlagen, in der Zuführeinrichtung zur Zellaufschlussvorrichtung wenigstens eine Störstoff-Erkennungseinrichtung für Störstoffe in der Biomasse, beispielsweise für Metallstücke, Steine, etc. vorzusehen. Bei einer Störstofferkennung kann dann die oben genannte Stellklappe zum Bypassweg und/oder die Transportrichtung für das dritte Fördermittel umgestellt werden, wobei dieser Vorgang vorzugsweise automatisiert ist.

Um entsprechend die Leistungsaufnahme insbesondere eines Elektroantriebs für die Zellaufschlussvorrichtung auf einem geeigneten Niveau zu vergleichmäßigen, wird mit Anspruch 10 eine Regelung der Extruder-Zuführmenge über einen Zuführregler vorgeschlagen, wobei diese Regelung auf eine gesteuert drehangetriebene Stopfschnecke wirkt. Dazu wird die Leistungsaufnahme als Istwert gemessen und beispielsweise bei einer vorgegebenen bzw. tendenziell abnehmenden Leistungsaufnahme die Stopfschneckendrehzahl über die Zuführregelung für eine größere Biomassenzufuhr erhöht. Diese Regelung ist zweckmäßig über geeignete Regelparameter für ein schnelleres Regelverhalten im Vergleich zur Düsendurchmesserregelung der Auslassöffnung ausgelegt.

Hinsichtlich des Verfahrens wird die Aufgabe mit den Merkmalen des Anspruchs 11 gelöst. Die sich hierdurch ergebenden Vorteile wurden bereits zuvor in Verbindung mit den Vorteilen für die Fermenter-Beschickungsanlage erörtert.

Anhand einer Zeichnung wird eine mögliche Ausführungsform der Erfindung beispielhaft näher erläutert.

Es zeigen:
- Fig. 1: eine perspektivische Ansicht einer Fermenter-Beschickungsanlage,
- Fig. 2: eine Draufsicht auf die Fermenter-Beschickungsanlage nach Fig. 1, und
- Fig. 3: einen Zellaufschlussextruder als Doppelschneckenextruder.

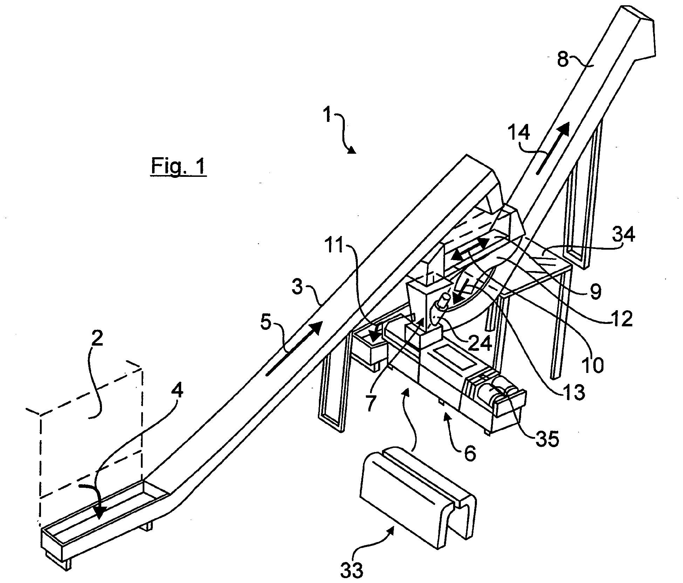

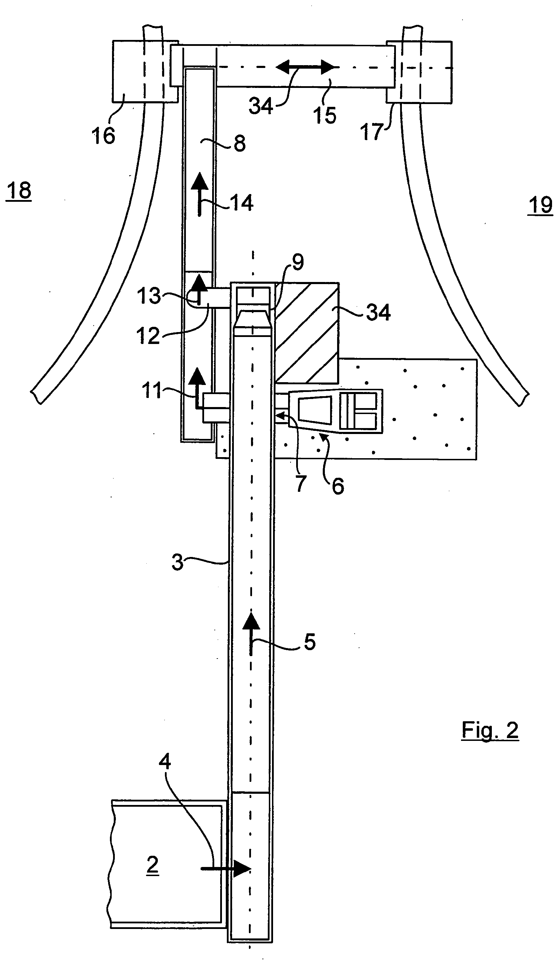

In den Fig. 1 und 2 ist in perspektivischer Darstellung und in einer Draufsicht eine Fermenter-Beschickungsanlage 1 gezeigt, wobei einer (nicht dargestellten) Biomassenlagerstelle als Fahrsilo ein nur schematisch angegebener Dosierer 2 nachgeordnet ist.

Vom Dosierer 2 wird gewichtsgesteuert Biomasse einem ersten Bandförderer 3 zugeführt (Pfeil 4), mit dem die Biomasse nach oben gefördert wird (Pfeil 5), und zwar in einen Bereich oberhalb eines eine Zellaufschlussvorrichtung bildenden Zellaufschlussextruders 6. Dort ist unter dem oberen Ende des ersten Bandförderers 3 und über einer Zuführeinrichtung 7 des Zellaufschlussextruders 6 ein mit einem zweiten Bandförderer 8 unmittelbar oder mit der Zuführeinrichtung 7 zusammenwirkender, dritter Bandförderer 9 angebracht. Dazu kann der dritte Bandförderer 9 in der Förderrichtung umgesteuert werden (Doppelpfeil 10), so dass (im Normalbetrieb) Biomasse, die vom ersten Bandförderer auf den dritten Bandförderer transportiert wird, über die Zuführeinrichtung 7 dem Zellaufschlussextruder 6 und nach dem dortiger Aufbereitung dem zweiten Bandförderer 8 zugeführt wird (Pfeil 11). Bei einer Umsteuerung der Laufrichtung des dritten Bandförderers 9, beispielsweise nach einer automatisierten Erkennung von Störstoffen im Förderweg zum Zellaufschlussextruder 6, gelangt Biomasse über ein Förderrohr 12 in einem Direktweg als Bypass durch freien Fall in den Bereich des Bandanfangs des zweiten Bandförderers 8 (Pfeil 13).

Mit dem zweiten Bandförderer 8 wird aufbereitete Biomasse aus dem Zellaufschlussextruder 6 (Pfeil 11) oder Biomasse über den Direktweg (Pfeil 13) nach oben transportiert (Pfeil 14), und zwar bis über einen querverlaufenden vierten Bandförderer 15, der zwischen zwei Fermenter-Beschickungsanschlüssen 16, 17 verläuft, wobei jeweils nur ein Teilabschnitt einer Fermenterwand der zugeordneten nebeneinanderliegenden Fermenter 18, 19 dargestellt ist (siehe Fig. 2). Der vierte Bandförderer 15 kann in seiner Laufrichtung umgesteuert werden (Doppelfpfeil 34), so dass je nach Laufrichtung der Fermenter 18 oder der Fermenter 19 mit Biomasse beschickt wird.

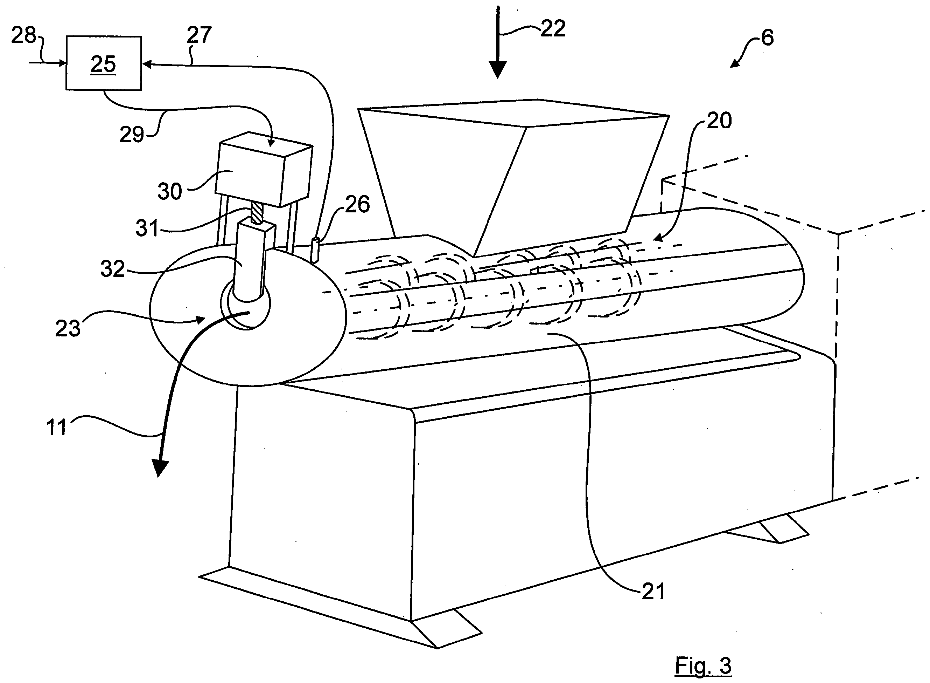

In Fig. 3 ist schematisch der vordere Teil des Zellaufschlussextruders 6 als Doppelschneckenextruder dargestellt. Dabei sind die zusammenwirkenden Doppelschnecken 20 in einem engen Schneckengehäuse 21 so angetrieben und angeordnet, dass zugeführte Biomasse (Pfeil 22) bei hohem Druckaufbau und starker Temperaturerhöhung gegen und durch eine Auslassdüse 23 nach außen gefördert werden (Pfeil 11), wo sich die zerfaserte und zerfranste Biomasse unter Zellaufschluss wieder entspannt und abkühlt.

Der Aufbau der Zuführeinrichtung 7 (in Fig. 3 noch nicht montiert) ist aus Fig. 1 ersichtlich und besteht hier beispielhaft aus einem Trichter mit zugeordneter Stopfschnecke 24. Die Stopfschnecke 24 ist dabei geregelt drehangetrieben, so dass die Biomassezufuhr (Pfeil 22) zum Zellaufschlussextruder 6 mengenmäßig so zudosiert wird, dass die Elektroantriebsmotoren 35 für die Doppelschnecken 20 mit weitgehend gleicher Leistung, zweckmäßig in einem oberen effektiven Leistungsbereich betrieben werden.

Der Durchmesser der Auslassdüse 23 ist hier mit einem Durchmesserregler 25 temperaturgeregelt ausgeführt, da für den Zellaufschluss eine Zelltemperatur der Biomassen von wenigstens 65°C im Schneckengehäuse 21 vor der Auslassdüse 23 erreicht werden soll. Somit ist in diesem Bereich ein Temperatursensor 26 als Temperatur-Istwertgeber angeordnet, dessen Istwertsignal dem Durchmesserregler 25 zugeführt wird (Pfeil 27). Als Sollwert (Pfeil 28) ist am Durchmesserregler 25 ein Temperaturwert von zum Beispiel 70°C eingestellt, der mit dem Temperatur-Istwert verglichen wird. Bei einer Regelabweichung gibt der Durchmesserregler 25 ein Stellsignal (Pfeil 29) an einen Stellmotor 30 ab, der mittels eines Spindeltriebs 31 einen Schieber 32 in der Auslassöffnung der Auslassdüse 23 verstellt dergestalt, dass bei einem Absinken der Isttemperatur unter die eingestellte Solltemperatur der Schieber 32 zur Durchmesserverkleinerung in die Auslassöffnung der Auslassdüse 23 weiter eingeschoben wird.

Da der Zellaufschlussextruder 6 als Bestandteil der relativ großen Fermenter-Beschickungsanlage 1 im Freien aufzustellen ist und eine hohe Temperatur vor der Auslassdüse 23 im Bereich der Doppelschnecke 20 für den Zellaufschluss erforderlich ist, wird insbesondere dieser Bereich wärmegedämmt ausgeführt. Dazu werden die in Fig. 1 dargestellten Dämmhalbschalen 33 aus Dämmmaterial mit den erforderlichen Ausschnitten für die Zuführeinrichtung 7 auf das Schneckengehäuse 21 oder gegebenenfalls den gesamten Extruder zu dessen Wärmedämmung aufgesetzt. Dadurch wird bei einem Temperaturwechsel der Extruderbetrieb vergleichmäßigt und die Regelung stabilisiert, wobei auch der Energiebedarf des Zellaufschlussextruders 6 im Vergleich zu einer fehlenden Wärmedämmung insgesamt vorteilhaft reduziert wird. Als weiterer Witterungsschutz kann der Extruderbereich noch überdacht werden (nicht dargestellt). Zudem kann zu Montage- und Wartungszwecke auf der Höhe des dritten Bandförderers 9 eine über (nicht dargestellte) Leitern zugängliche Plattform 34 angebracht sein.

## Patentansprüche

1. Fermenter-Beschickungsanlage für vergärbare Biomasse einer Biogasanlage,
mit einer Förder- und Aufbereitungseinrichtung zwischen einer Biomasselagerstelle und wenigstens einem Fermenter, die wenigstens ein Fördermittel für die dem Fermenter (18, 19) zuzuführende Biomasse aufweist, wobei in die Förder- und Aufbereitungseinrichtung weiter eine Zellaufschlussvorrichtung (6) für Biomasse integriert ist, über die zumindest ein Teil der zum Fermenter (18, 19) förderbaren Biomasse geleitet (5, 10, 11, 14, 34) und nach dem Zellaufschluss dem Fermenter (18, 19) zugeführt wird,
**dadurch gekennzeichnet,**
**dass** der Durchmesser einer Auslassöffnung (23) der Zellaufschlussvorrichtung (6) durch ein gesteuert verstellbares Stellglied (32) veränderbar ist,
**dass** ein Temperatursensor (26) vorgesehen ist, mittels dem die Temperatur in einem vorgegebenen Bereich der Zellaufschlussvorrichtung (6) erfasst wird, wobei ein Durchmesser-Regler (25) vorgesehen ist, dem das Ausgangssignal des Temperatursensors (26) als Temperatur-Istwert (27) zuführbar ist, wobei der Istwert mit einem vorgegebenen Sollwert (28) verglichen wird und das Stellglied (32) bei einem Absinken des Istwerts unter den Sollwert für eine vorgegebene Reduzierung des Auslassöffnungs-Durchmessers angesteuert wird, und/oder dass ein Drucksensor vorgesehen ist, mittels dem der Druck in einem vorgegebenen Bereich der Zellaufschlussvorrichtung (6) erfasst wird, wobei ein Durchmesser-Regler (25) vorgesehen ist, dem das Ausgangssignal des Drucksensors als Druck-Istwert zugeführt wird, wobei der Istwert mit einem vorgegebenen Sollwert (28) verglichen wird und das Stellglied (32) bei einem Absinken des Istwerts unter den Sollwert für eine vorgegebene Reduzierung des Auslassöffnungs-Durchmessers angesteuert wird.

2. Fermenter-Beschickungsanlage nach Anspruch 1, **dadurch gekennzeichnet, dass** der Temperatursensor (26) und/oder der Drucksensor in der Zellaufschlussvorrichtung (6) unmittelbar vor oder in der Auslassöffnung (23), insbesondere unmittelbar vor oder in einer Auslassdüse eines Zellaufschlussextruders, angeordnet ist.

3. Fermenter-Beschickungsanlage nach Anspruch 1 oder 2, **dadurch gekennzeichnet, dass** der Förder- und Aufbereitungseinrichtung eine Zerkleinerungs- und Mischeinrichtung vorgeschaltet ist, die vorzugsweise mit einer Dosiervorrichtung (2) eine Baueinheit bildet.

4. Fermenter-Beschickungsanlage nach einem der Ansprüche 1 bis 3, **dadurch gekennzeichnet, dass** die Zellaufschlussvorrichtung für einen Außeneinsatz wärmegedämmt ausgeführt ist durch eine unmittelbare Wärmedämmung in der Art einer Wärmedämm-Umhüllung (33) oder durch eine mittelbare Wärmedämmung in der Art einer Wärmedämm-Einhausung.

5. Fermenter-Beschickungsanlage nach einem der Ansprüche 1 bis 4, **dadurch gekennzeichnet, dass** die Zellaufschlussvorrichtung ein Zellaufschlussextruder (6) mit Schneckenwellen (20), vorzugsweise ein Doppelschneckenextruder, ist, der eine Zuführeinrichtung (7) und eine Auslassdüse als Auslassöffnung (23) aufweist.

6. Fermenter-Beschickungsanlage nach Anspruch 5, **dadurch gekennzeichnet, dass** wenigstens im Schneckenbereich außen eine Wärmedämmung (33) angebracht ist.

7. Fermenter-Beschickungsanlage nach einem der Ansprüche 1 bis 6, **dadurch gekennzeichnet,**
**dass** ein erstes Fördermittel (3), insbesondere ein erster Bandförderer von einer Dosiervorrichtung (2) in einen Bereich oberhalb der Zellaufschlussvorrichtung (6) führt,
**dass** ein zweites Fördermittel (8), insbesondere ein zweiter Bandförderer, ausgehend von einem Bereich unter einer Auslassöffnung der Zellaufschlussvorrichtung (6), bevorzugt unter eine Auslassdüse (23) eines Zellaufschluss-Extruders (6), direkt oder indirekt zum Fermenter-Beschickungsanschluss (16, 17) führt,
**dass** das Förderende des ersten Fördermittels (3) oberhalb einer Zuführeinrichtung (7) der Zellaufschlussvorrichtung (6) liegt, wobei die Zuführeinrichtung (7) einen Zuführtrichter mit einer motorisch antreibbaren Stopfschnecke (24), eine Stellklappe und einen Bypassweg zum zweiten Fördermittel (8) aufweist, so dass je nach Position der Stellklappe Biomasse der Stopfschnecke (24) für einen Zellaufschluss oder über den Bypassweg unmittelbar dem zweiten Fördermittel (8) für einen Transport in den Fermenter zugeführt wird.

8. Fermenter-Beschickungsanlage nach Anspruch 7, **dadurch gekennzeichnet, dass** ein drittes Fördermittel (9), insbesondere ein dritter Bandförderer, vorgesehen ist, wobei das Förderende des ersten Fördermittels (3) in einem mittleren Bereich über dem dritten Fördermittel (9) liegt und mit dem dritten Fördermittel (9) in einer Transportrichtung (10) Biomasse zur Zuführeinrichtung (7) der Zellaufschlussvorrichtung (6) und in der anderen Transportrichtung (10) über einen Direktweg (12) unmittelbar zum zweiten Fördermittel (8) transportierbar ist, wobei gegebenenfalls die Stellklappe und der dieser zugeordnete Bypassweg entfallen.

9. Fermenter-Beschickungsanlage nach Anspruch 7 oder Anspruch 8, **dadurch gekennzeichnet,**
**dass** der Zuführeinrichtung (7) der Zellaufschlussvorrichtung (6) wenigstens eine Störstoff-Erkennungseinrichtung für Störstoffe in der Biomasse zugeordnet ist, und
**dass** bei einer Störstofferkennung die Stellklappe zum Bypassweg und/oder die Transportrichtung (10) für das dritte Fördermittel (9) umgestellt, vorzugsweise automatisch umgesteuert wird.

10. Fermenter-Beschickungsanlage nach einem der Ansprüche 1 bis 9, **dadurch gekennzeichnet, dass** für eine gesteuert motorisch mittels eines Elektroantriebs oder Hydraulikantriebs oder Pneumatikantriebs drehantreibbare Stopfschnecke (24) in der Zuführeinrichtung (7) einer Zellaufschlussvorrichtung (6) ein Zuführ-Regler vorgesehen ist, mit einem Leistungsmesser für die von dem oder den Schneckenantriebsmotoren (35) aufgenommen elektrischen Leistung als Leistungs-Istwertgeber, einem Leistungs-Sollwertgeber und dem Drehantrieb der Stopfschnecke (24) als Stellglied, wobei insbesondere bei einer vorgegebenen, gegenüber dem eingestellten Leistungs-Sollwert abnehmenden Leistungsaufnahme durch Erhöhung der Stopfschnecken-Drehzahl mehr Biomasse dem Zellaufschlussextruder (6) zuführbar ist.

11. Verfahren zum Betreiben einer Fermenter-Beschickungsanlage nach einem der Ansprüche 1 bis 10, **dadurch gekennzeichnet, dass** ein Temperatur-Sollwert von mindestens 40°C, höchst bevorzugt von mindestens 60°C vorgegeben wird, so dass bei einem Unterschreiten dieses vorgegebenen Temperatur-Sollwertes der Auslassöffnungs-Durchmesser in einem vorgegebenen Maße, bevorzugt kennfeldgesteuert, so reduziert wird, dass sich der Temperatur-Istwert auf den vorgegebenen Temperatur-Sollwert einstellt und/oder einregelt.

## Claims

1. Fermenter feed system for fermentable biomass of a biogas system, comprising a delivery and treatment device between a biomass storage site and at least one fermenter, said device comprising at least one delivery means for the biomass to be fed to the fermenter (18, 19), wherein furthermore a cell breakdown apparatus (6) for biomass, via which at least part of the biomass that can be delivered to the fermenter (18, 19) is conducted (5, 10, 11, 14, 34) and fed to the fermenter (18, 19) after the cell breakdown, is integrated in the delivery and treatment device,
**characterized**
**in that** the diameter of an outlet opening (23) of the cell breakdown apparatus (6) can be varied by an actuating member (32) that is adjustable in a controlled manner,
**in that** a temperature sensor (26) is provided, by means of which the temperature in a predetermined region of the cell breakdown apparatus (6) is sensed, wherein a diameter regulator (25) is provided, to which the output signal of the temperature sensor (26) can be fed as an actual temperature value (27), wherein the actual value is compared with a predetermined setpoint value (28) and the actuating member (32) is activated if the actual value falls below the setpoint value for a predetermined reduction of the diameter of the outlet opening,
and/or in that a pressure sensor is provided, by means of which the pressure in a predetermined region of the cell breakdown apparatus (6) is sensed, wherein a diameter regulator (25) is provided, to which the output signal of the pressure sensor is fed as an actual pressure value, wherein the actual value is compared with a predetermined setpoint value (28) and the actuating member (32) is activated if the actual value falls below the setpoint value for a predetermined reduction of the diameter of the outlet opening.

2. Fermenter feed system according to Claim 1, **characterized in that** the temperature sensor (26) and/or the pressure sensor is arranged in the cell breakdown apparatus (6) directly in front of or in the outlet opening (23), in particular directly in front of or in an outlet die of a cell breakdown extruder.

3. Fermenter feed system according to Claim 1 or 2, **characterized in that** the delivery and preparation device is preceded by a comminuting and mixing device, which preferably forms a structural unit with a metering apparatus (2).

4. Fermenter feed system according to one of Claims 1 to 3, **characterized in that** the cell breakdown apparatus is of a thermally insulated design for outdoor use, as a result of a direct heat insulation in the manner of a heat-insulating casing (33) or as the result of an indirect heat insulation in the manner of a heat-insulating enclosure.

5. Fermenter feed system according to one of Claims 1 to 4, **characterized in that** the cell breakdown apparatus has a cell breakdown extruder (6) with screw shafts (20), preferably a twin-screw extruder, which has a feeding device (7) and an outlet die as the outlet opening (23).

6. Fermenter feed system according to Claim 5, **characterized in that**, at least in the region of the screw, a heat insulation (33) is provided on the outside.

7. Fermenter feed system according to one of Claims 1 to 6, **characterized in that** a first delivery means (3), in particular a first belt conveyor, leads from a metering apparatus (2) into a region above the cell breakdown apparatus (6),
**in that** a second delivery means (8), in particular a second belt conveyor, leads, starting from a region below an outlet opening of the cell breakdown apparatus (6), preferably under an outlet die (23) of a cell breakdown extruder (6), directly or indirectly to the fermenter feed connection (16, 17),
**in that** the delivery end of the first delivery means (3) lies above a feeding device (7) of the cell breakdown apparatus (6), wherein the feeding device (7) has a feeding hopper with a motor-drivable stuffing screw (24), a control damper and a bypass path to the second delivery means (8), so that, depending on the position of the control damper, biomass is fed to the stuffing screw (24) for cell breakdown or is fed via the bypass path directly to the second delivery means (8) for being transported into the fermenter.

8. Fermenter feed system according to Claim 7, **characterized in that** a third delivery means (9), in particular a third belt conveyor, is provided, wherein the delivery end of the first delivery means (3) lies in a central region above the third delivery means (9) and biomass can be transported by the third delivery means (9) in one transporting direction (10) to the feeding device (7) of the cell breakdown apparatus (6) and in the other transporting direction (10) via a direct path (12) directly to the second delivery means (8), wherein the control damper and the bypass path assigned to it are optionally omitted.

9. Fermenter feed system according to Claim 7 or Claim 8, **characterized in that** the feeding device (7) of the cell breakdown apparatus (6) is assigned at least one impurity detection device for impurities in the biomass, and
**in that**, in the event of an impurity being detected, the control damper to the bypass path and/or the transporting direction (10) for the third delivery means (9) is changed over, preferably is automatically reversed.

10. Fermenter feed system according to one of Claims 1 to 9, **characterized in that** a feed regulator is provided for a stuffing screw (24) that can be rotationally motor-driven by means of an electric drive, a hydraulic drive or a pneumatic drive in the feeding device (7) of a cell breakdown apparatus (6), having a power meter for the electrical power consumed by the screw drive motor or motors (35) as a power actual-value sensor, a power setpoint generator and the rotary drive of the stuffing screw (24) as an actuating member, wherein, in particular when there is a predetermined decreasing power consumption with respect to the setpoint power value that has been set, more biomass can be fed to the cell breakdown extruder (6) by increasing the rotational speed of the stuffing screw.

11. Method for operating a fermenter feed system according to one of Claims 1 to 10, **characterized in that** a setpoint temperature value of at least 40°C, at most preferably of at least 60°C, is predetermined, so that, when the temperature falls below this predetermined setpoint temperature value, the diameter of the outlet opening is reduced to a predetermined extent, preferably under the control of a characteristic map, such that the temperature actual value is set and/or regulated to the predetermined setpoint temperature value.

## Revendications

1. Équipement d'alimentation de fermenteur pour une biomasse fermentable d'un système de biogaz,
comprenant un dispositif de transport et de préparation entre un lieu de stockage de biomasse et au moins un fermenteur, lequel possède au moins un moyen de transport pour la biomasse à acheminer au fermenteur (18, 19), un arrangement de désagrégation cellulaire (6) pour la biomasse étant en plus intégré dans le dispositif de transport et de préparation, par le biais duquel au moins une partie de la biomasse pouvant être transportée au fermenteur (18, 19) est dirigée (5, 10, 11, 14, 34) puis acheminée au fermenteur (18, 19) après la désagrégation cellulaire,
**caractérisé en ce**
**que** le diamètre d'une ouverture de sortie (23) de l'arrangement de désagrégation cellulaire (6) peut être modifié par un actionneur (32) positionnable de manière commandée,
**qu'**une sonde de température (26) est présente, au moyen de laquelle la température est détectée dans une zone prédéfinie de l'arrangement de désagrégation cellulaire (6), un régulateur de diamètre (25) étant présent, auquel le signal de sortie de la sonde de température (26) peut être acheminé en tant que valeur réelle de la température (27), la valeur réelle étant comparée à une valeur de consigne (28) prédéfinie et l'actionneur (32) étant commandé pour une réduction prédéfinie du diamètre de l'ouverture de sortie en cas de chute de la valeur réelle au-dessous de la valeur de consigne, et/ou en ce qu'un capteur de pression est présent, au moyen duquel la pression est détectée dans une zone prédéfinie de l'arrangement de désagrégation cellulaire (6), un régulateur de diamètre (25) étant présent, auquel le signal de sortie du capteur de pression est acheminé en tant que valeur réelle de la pression, la valeur réelle étant comparée à une valeur de consigne (28) prédéfinie et l'actionneur (32) étant commandé pour une réduction prédéfinie du diamètre de l'ouverture de sortie en cas de chute de la valeur réelle au-dessous de la valeur de consigne.

2. Équipement d'alimentation de fermenteur selon la revendication 1, **caractérisé en ce que** la sonde de température (26) et/ou le capteur de pression dans l'arrangement de désagrégation cellulaire (6) est disposé immédiatement avant ou dans l'ouverture de sortie (23), notamment immédiatement avant ou dans une buse de sortie d'une extrudeuse de désagrégation cellulaire.

3. Équipement d'alimentation de fermenteur selon la revendication 1 ou 2, **caractérisé en ce qu'**un dispositif de fragmentation et de mélange est monté en amont du dispositif de transport et de préparation, lequel, avec un arrangement de dosage (2), forme de préférence une unité modulaire.

4. Équipement d'alimentation de fermenteur selon l'une des revendications 1 à 3, **caractérisé en ce que** l'arrangement de désagrégation cellulaire est réalisé isolé thermiquement pour une utilisation à l'extérieur, par une isolation thermique directe sous la forme d'une enveloppe d'isolation thermique (33) ou par une isolation thermique indirecte sous la forme d'une enceinte d'isolation thermique.

5. Équipement d'alimentation de fermenteur selon l'une des revendications 1 à 4, **caractérisé en ce que** l'arrangement de désagrégation cellulaire est une extrudeuse de désagrégation cellulaire (6) munie d'arbres à vis sans fin (20), de préférence une extrudeuse à double vis sans fin, qui possède un dispositif d'acheminement (7) et une buse de sortie en tant qu'ouverture de sortie (23).

6. Équipement d'alimentation de fermenteur selon la revendication 5, **caractérisé en ce qu'**au moins dans la zone de vis sans fin est montée à l'extérieur une isolation thermique (33).

7. Équipement d'alimentation de fermenteur selon l'une des revendications 1 à 6, **caractérisé en ce**
**qu'**un premier moyen de transport (3), notamment un premier convoyeur à bande mène depuis un arrangement de dosage (2) dans une zone au-dessus de l'arrangement de désagrégation cellulaire (6),
en ce qu'un deuxième moyen de transport (8), notamment un deuxième convoyeur à bande, mène depuis une zone sous une ouverture de sortie de l'arrangement de désagrégation cellulaire (6), de préférence sous une buse de sortie (23) d'une extrudeuse de désagrégation cellulaire (6), directement ou indirectement au raccord d'alimentation de fermenteur (16, 17),
en ce que l'extrémité de transport du premier moyen de transport (3) se trouve au-dessus d'un dispositif d'amenée (7) de l'arrangement de désagrégation cellulaire (6), le dispositif d'amenée (7) possédant un entonnoir d'amenée muni d'une vis sans fin de gavage (24) à entraînement motorisé, un clapet de commande et un trajet de contournement vers le deuxième moyen de transport (8), de sorte que suivant la position du clapet de commande, la biomasse est acheminée à la vis sans fin de gavage (24) pour une désagrégation cellulaire ou par le biais du trajet de contournement directement au deuxième moyen de transport (8) pour un transport dans le fermenteur.

8. Équipement d'alimentation de fermenteur selon la revendication 7, **caractérisé en ce qu'**un troisième moyen de transport (9), notamment un troisième convoyeur à bande, est présent, l'extrémité de transport du premier moyen de transport (3) se trouvant dans une zone centrale au-dessus du troisième moyen de transport (9) et la biomasse pouvant être transportée avec le troisième moyen de transport (9) dans une direction de transport (10) vers le dispositif d'amenée (7) de l'arrangement de désagrégation cellulaire (6) et dans l'autre direction de transport (10) par un trajet direct (12) directement vers le deuxième moyen de transport (8), le clapet de commande et le trajet de contournement qui lui est associé étant éventuellement supprimés.

9. Équipement d'alimentation de fermenteur selon la revendication 7 ou la revendication 8, **caractérisé en ce**
**qu'**au moins un dispositif de reconnaissance de substance perturbatrice pour des substances perturbatrices dans la biomasse est associé au dispositif d'amenée (7) de l'arrangement de désagrégation cellulaire (6) et
en ce qu'en cas de reconnaissance d'une substance perturbatrice, le clapet de commande est déplacé, de préférence permuté automatiquement, vers le trajet de contournement et/ou la direction de transport (10) pour le troisième moyen de transport (9) est modifiée, de préférence permutée automatiquement.

10. Équipement d'alimentation de fermenteur selon l'une des revendications 1 à 9, **caractérisé en ce que** dans le dispositif d'amenée (7) d'un l'arrangement de désagrégation cellulaire (6), pour une vis sans fin de gavage (24) qui peut être entraînée en rotation de manière motorisée et commandée au moyen d'un mécanisme d'entraînement électrique ou d'un mécanisme d'entraînement hydraulique ou d'un mécanisme d'entraînement pneumatique, se trouve un régulateur d'amenée comprenant un appareil de mesure de puissance sous la forme d'un transmetteur de valeur réelle de puissance pour la puissance électrique absorbée par le ou les moteurs d'entraînement de vis sans fin (35), un transmetteur de valeur de consigne de puissance et le mécanisme d'entraînement en rotation de la vis sans fin de gavage (24) en tant qu'actionneur, un volume plus élevé de biomasse pouvant être acheminé à l'extrudeuse de désagrégation cellulaire (6), notamment en présence d'une consommation de puissance prédéfinie qui baisse par rapport à la valeur de consigne de puissance réglée, en augmentant la vitesse de rotation de la vis sans fin de gavage.

11. Procédé pour faire fonctionner un équipement d'alimentation de fermenteur selon l'une des revendications 1 à 10, **caractérisé en ce qu'**une valeur de consigne de température d'au moins 40 °C, de préférence au maximum d'au moins 60 °C est prédéfinie, de sorte que lors d'un passage en dessous de cette valeur de consigne de température prédéfinie, le diamètre de l'ouverture de sortie est réduit dans une cote prédéfinie, de préférence de manière commandée par un diagramme caractéristique, de sorte que la valeur réelle de la température est réglée et/ou est asservie à la valeur de consigne de température prédéfinie.
